# EUROPÄISCHE PATENTSCHRIFT

(11) **EP 1 206 970 B1**
(45) Veröffentlichungstag und Bekanntmachung des Hinweises auf die Patenterteilung: **22.03.2006**
(21) Anmeldenummer: 01125198.0
(22) Anmeldetag: 24.10.2001
(51) Int. Cl.: B01L 9/00, C12M 1/22, B65D 85/62

(54) **Kassetten-Anordnung zur Aufnahme von Schachteln o.dgl., insbesondere Petrischalen**
Cassette for holding boxes, especially Petri dishes
Cassette pour ranger des boîtes, en particulier des boîtes de Petri

(30) Priorität: 03.11.2000 CH 21442000
(43) Veröffentlichungstag der Anmeldung: 22.05.2002
(73) Patentinhaber: Zingre, Hans, 8634 Hombrechtikon ZH (CH)
(72) Erfinder: Zingre, Hans, 8634 Hombrechtikon ZH (CH)
(74) Vertreter: Petschner, Goetz

(56) Entgegenhaltungen:
- EP-A- 1 018 544
- DE-U- 9 300 105
- US-A- 4 170 861
- US-A- 5 706 943
- US-A- 5 747 333

## Beschreibung

Die vorliegende Erfindung betrifft eine Kassetten-Anordnung zur Aufnahme von Schachteln o.dgl., insbesondere Petrischalen.

In vielen Bereichen der Technik werden hochempfindliche Güter, wie Chips, etwa zur Zwischenlagerung eingeschachtelt. Vor allem aber in mikrobiologischen Laboratorien werden für den qualitativen und quantitativen Nachweis von Mikroorganismen sowie in zeilbiologischen Labors für die Zellzüchtung sogenannte Petrischalen in Form von flachen Glasschälchen mit Deckel verwendet, die man dann aus Platzgründen meistens bis zu 20 oder mehr Stück aufeinander stapelt. Solche relativ hohen Stapel sind jedoch sehr unstabil und können sehr leicht umfallen, sodass die Deckel der Schalen wegfallen und sich somit beispielsweise Kontaminationen im Labor ausbreiten können, was fatale Folgen haben kann.

Durch die US-PS 5'706'943 ist zwar bereits eine Kassetten-Anordnung zur Aufnahme von CD-Bild- und Tonträger bekannt geworden, mit einem stapelbar ausgebildeten, flächig-kastenförmigen Kassettengehäuse mit einer stirnseitigen schlitzförmigen Öffnung, durch welche eine Schublade zugänglich ist zum Einlegen oder Entnehmen einer CD, sowie mit an der gegenüberliegenden Stirnseite angeordnete fingerbetätigbare Mittel zum wenigstens teilweise Herausschieben der Schublade, wobei das Kassettengehäuse zum gesicherten Stapeln mehrerer Kassetten planseitige Stapelmittel, hier in Gegenbohrungen eingreifende Zapfen aufweist.

Von diesem Stand der Technik ausgehend, ist es nun Aufgabe der vorliegenden Erfindung, eine Kassetten-Anordnung zur Aufnahme von Schachteln o.dgl., insbesondere Petrischalen zu schaffen, die, unkompliziert im Aufbau, eine sichere Handhabung und den sicheren Transport von allen gängigen 80 -100mm Standard-Petrischalen u. dgl. gestattet. Die Kassetten-Anordnung soll dabei ein gesichertes Stapeln sowie eine problemlose schubladenfreie Entnahme einzelner Schachteln resp. Petrischalen aus dem Stapel erlauben.

Dies wird erfindungsgemäss zunächst dadurch erreicht, dass die fingerbetätigbaren Mittel zum wenigstens teilweise Herausschieben einer Schale durch die Öffnung des Kassettengehäuses mindestens eine, seitlich über Schamiermittel nach innen einschwenkbare Klinke umfassen.

Durch diese Massnahmen wird bei solchen sogenannten Dispensem eine sichere Handhabung und Transport von Petrischalen u. dgl. gewährleistet. Die Kassetten-Anordnungen gestatten zudem ein gesichertes Stapeln sowie eine problemlose Entnahme einzelner Schachteln resp. Petrischalen aus dem Stapel.

Bei einer bevorzugten Ausgestaltung der erfindungsgemässen Kassetten-Anordnung können der Öffnung des Kassettengehäuses gegenüberliegend zwei eckseitige, je nach innen einschwenkbare Klinken vorgesehen werden.

Zur Sicherung des Deckels der eingebrachten Petrischale und zu deren eigenen Sicherung in der Kassette ist es zudem zweckmässig, wenn am Kassettengehäuse mindestens auf einer Planseite ein in das Innere des Gehäuses wirksamer Federteil angeformt ist.

Vorzugsweise besteht die Kassetten-Anordnung aus einem hitzebeständigen und gegen agressive Stoffe resistenten, vorzugsweise wenigstens teilweise transparenten Kunststoff, wobei eine einstückige Ausbildung zweckmässig ist, bei der die einschwenkbaren Klinken Wandungsteile des Kassettengehäuses bilden und die Schamiermittel Filmschamiere sind. Durch diese Ausbildung ist es möglich, auf die Anordnung von separaten Federmitteln und Auswerfer zu verzichten.

Beispielsweise Ausführungsformen des Erfindungsgegenstandes sind nachfolgend anhand der Zeichnung näher erläutert. Es zeigen:
- Fig. 1: in schaubildartiger Darstellung eine erfindungsgemässe Kassetten-Anordnung;
- Fig. 2a u. 2b: in Draufsicht in 2 Handhabungsfunktionen eine Kassetten-Anordnung gemäss Fig. 1 mit eingebrachter Petrischale;
- Fig. 3: die Kassetten-Anordnung gemäss Fig. 2a in Seitenansicht und
- Fig.4: in Frontansicht einen Stapel von Kassetten-Anordnungen gemäss Fig. 1.

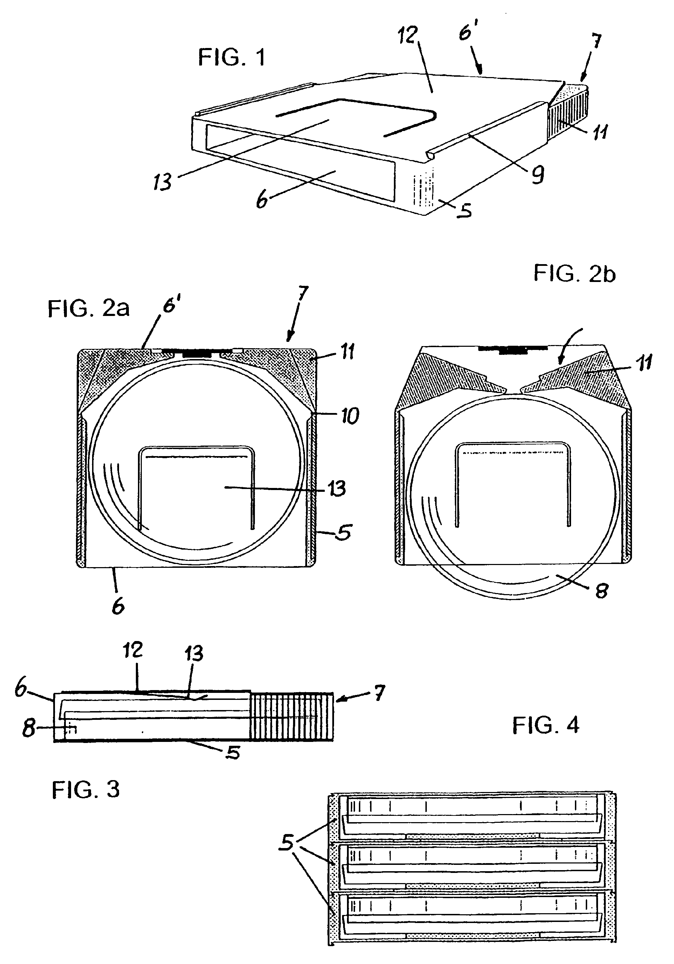

Aus den Fig. 1 bis 3 ist eine Kassetten-Anordnung zur Aufnahme von Schachteln o.dgl., insbesondere Petrischalen entnehmbar, welche sich erfindungsgemäss auszeichnet durch ein stapelbar ausgebildetes, flächig-kastenförmiges Kassettengehäuse 5 mit einer stimseitigen schlitzförmigen Öffnung 6 zum Einlegen oder Entnehmen einer Schachtel oder Schale 8 sowie mit an der gegenüberliegenden Stirnseite 6' angeordneten fingerbetätigbaren Mittel 7 zum wenigstens teilweise Herausschieben der Schale 8 durch die Öffnung 6 des Kassettengehäuses 5.

Zum gesicherten Stapeln mehrerer solcher Kassetten weist das Kassettengehäuse 5 planseitige Stapelmittel, beispielsweise Stapelränder 9 in Form von Schwalbenschwanz-Profilen oder Druckknopfanordnungen o.dgl. auf.

Zum wenigstens teilweise Herausschieben einer eingebrachten Schale 8 durch die Öffnung 6 des Kassettengehäuses 5 umfassen die fingerbetätigbaren Mitteln 7 mindestens eine, seitlich über Scharniermittel 10 nach innen einschwenkbare Klinke 11, vorzugsweise jedoch zwei eckseitige Klinken.

Weiter ist am Kassettengehäuse 5 mindestens auf einer Planseite 12 ein in das Innere des Gehäuses 5 wirksamer Federteil 13 angeformt, welcher den Deckel einer eingebrachten Petrischale fixiert, wie insbesondere Fig. 3 veranschaulicht.

Vorzugsweise besteht die Kassetten-Anordnung aus einem hitzebeständigen und gegen agressive Stoffe resistenten, wenigstens teilweise transparenten Kunststoff, wobei dann die einschwenkbaren Klinken 11 Wandungsteile des Kassettengehäuses 5 bilden und die Schamiermittel 10 Filmschamiere sind.

Durch diese Massnahmen wird es möglich, eine Kassetten-Anordnung zur Aufnahme von Schachteln o.dgl., insbesondere Petrischalen zu schaffen, die eine sichere Handhabung und den Transport von allen gängigen 80 -100mm Standard-Petrischalen u. dgl. gestattet. Die Kassetten-Anordnung gestattet dabei ein gesichertes Stapeln sowie eine problemlose Entnahme einzelner Schachteln resp. Petrischalen aus dem Stapel.

Selbstverständlich sind im Rahmen der vorbeschriebenen Erfindung eine ganze Reihe von Modifikationen möglich, ohne dabei den Erfindungsgedanken zu verlassen.

Es wird Schutz beansprucht wie folgt:

## Patentansprüche

1. Kassetten-Anordnung zur Aufnahme von Schachteln o.dgl., insbesondere Petrischalen, bestehend aus einem stapelbar ausgebildeten, flächig-kastenförmigen Kassettengehäuse mit einer stimseitigen schlitzförmigen Öffnung zum Einlegen oder Entnehmen einer Schachtel oder Schale sowie mit an der gegenüberliegenden Stirnseite angeordneten fingerbetätigbaren Mittel zum wenigstens teilweise Herausschieben der Schale durch die Öffnung des Kassettengehäuses,
wobei das Kassettengehäuse zum gesicherten Stapeln mehrerer Kassetten planseitige Stapelmittel, beispielsweise in Gegenbohtungen eingreifende Zapfen o.dgl. aufweist.
**dadurch gekennzeichnet,**
**dass** die fingerbetätigbaren Mittel (7) zum wenigstens teilweise Herausschieben einer Schale (8) durch die Öffnung (6) des Kassettengehäuses (5) mindestens eine, seitlich über Schamiermittel (10) nach innen einschwenkbare Klinke (11) umfassen.

2. Kassetten-Anordnung nach Anspruch 1,
**dadurch gekennzeichnet,**
**dass** der Öffnung (6) des Kassettengehäuses (5) gegenüberliegend zwei eckseitige, je nach innen einschwenkbare Klinken (11) vorgesehen sind.

3. Kassetten-Anordnung nach Anspruch 1,
**dadurch gekennzeichnet,**
**dass** am Kassettengehäuse (5) mindestens auf einer Planseite (12) ein in das Innere des Gehäuses (5) wirksamer Federteil (13) angeformt ist.

4. Kassetten-Anordnung nach den Ansprüchen 1 bis 3,
**dadurch gekennzeichnet,**
**dass** diese aus einem hitzebeständigen und gegen agressive Stoffe resistenten Kunststoff besteht.

5. Kassetten-Anordnung nach den Ansprüchen 1 bis 4,
**gekennzeichnet durch**
eine einstückige Ausbildung, wobei die einschwenkbaren Klinken (11) Wandungsteile des Kassettengehäuses (5) bilden und die Scharniermittel (10) Filmschamiere sind.

## Claims

1. A cassette for holding boxes, especially petri dishes, comprising a shallow, box-shaped cassette housing which is designed to be stackable and has a slotted opening an the end face for inserting or removing a box or a dish, and with finger-operated means arranged an the opposite end face for pushing the dish at least partially out through the opening in the cassette housing, wherein the cassette housing has stacking means an the flat side, e.g., pushbutton arrangements or the like for secure stacking of multiple cassettes, **characterised in that**
the finger-operated means comprise at least one latch that can be pivoted inward at the side by hinge means for pushing the dish at least partially out through the opening in the cassette housing.

2. The cassette according to Claim 1, wherein two corner latches, each capable of pivoting inward, are provided opposite the opening in the cassette housing.

3. The cassette according to Claim 1, wherein a spring part which acts toward the interior of the housing is integrally molded an at least one flat side of the cassette housing.

4. The cassette according to Claims 1 to 3, whereby it is made of a heat-resistant plastic which is resistant to aggressive substances.

5. The cassette according to Claims 1 to 4, wherein
it is an one-piece design, where the pivotable latches form parts of the wall of the cassette housing, and the hinge means are film hinges.

## Revendications

1. Agencement de cassette pour la réception de boîtes ou analogues, en particulier de boîtes de Petri, constitué d'un boîtier de cassette en forme de caisson plat, réalisé d'une manière empilable, avec une ouverture en forme de fente au coté frontal pour insérer ou retirer une boîte, et avec des moyens actionnables par un doigt, disposés sur le côté frontal opposé, pour faire sortir par poussée la boîte au moins partiellement à travers l'ouverture du boîtier de cassette, où le boîtier de cassette, en vue d'un empilage assuré de plusieurs cassettes, présente des moyens d'empilage côté plan, par exemple, des tenons ou analogues s'engageant dans des contre-perçages,
**caractérisé en ce que** les moyens actionnables au doigt (7) pour faire sortir au moins partiellement une boîte (8) à travers l'ouverture (6) du boîtier de cassettes (5) comprennent au moins un cliquet (11) pouvant être amené à pivoter latéralement par des moyens de charnière (10) vers l'intérieur.

2. Agencement de cassette selon la revendication 1,
**caractérisé en ce que** sont prévus en face de l'ouverture (6) du boîtier de cassettes (5) deux cliquets côté coin, pouvant être amenés à pivoter chacun vers l'intérieur.

3. Agencement de cassette selon la revendication 1,
**caractérisé en ce qu'**il est rapporté par formage au boîtier de cassettes (5) au moins sur un côté plan (12) une partie élastique (13) agissant sur l'intérieur du boîtier (5).

4. Agencement de cassette selon les revendications 1 à 3,
**caractérisé en ce que** celui-ci est constitué d'un matériau synthétique résistant à la chaleur et résistant à des matières agressives.

5. Agencement de cassette selon les revendications 1 à 4,
**caractérisé par** une réalisation en une pièce, où les cliquets (11) aptes à être pivotés vers l'intérieur forment des parties de paroi du boîtier de cassettes (5), et les moyens de charnière (10) sont des charnières de feuille.
